# EUROPEAN PATENT APPLICATION

(11) **EP 1 022 275 A1**
(43) Date of publication of application: **26.07.2000**
(21) Application number: 00100252.6
(22) Date of filing: 18.01.2000
(51) Int. Cl.: C07D 303/16

(54) **Process for purifying glycidyl (Meth) acrylate**

(30) Priority: 20.01.1999 JP 1220099
(71) Applicant: MITSUBISHI GAS CHEMICAL COMPANY, INC., Tokyo 100-0005 (JP)
(72) Inventor: Tanaka, Kazumi, Mitsubishi Gas Chemical Co., Inc., Niigata-shi, Niigata-ken (JP); Kurose, Hideyuki, Mitsubishi Gas Chemical Co., Inc, Niigata-shi, Niigata-ken (JP); Higuchi, Hirofumi, Mitsubishi Gas Chemical Co.,Inc, Niigata-shi, Niigata-ken (JP)
(74) Representative: Gille Hrabal Struck Neidlein Prop Roos

(57) **Abstract**

A crude glycidyl (meth)acrylate is purified by contacting it with a resin having ion exchange groups represented by the following formula (I): 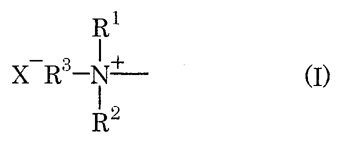 wherein R¹, R² and R³ are the same or different and are each H, HOC₂H₄, a C₁-C₁₀ alkyl group, a C₆-C₁₀ aryl group or a C₇-C₁₀ aralkyl group, and X is a halogen atom. During the contact with the resin, 1,3-dichloro-2-propanol contained in the crude glycidyl (meth)acrylate as impurity is converted to epichlorohydrin, which is readily separated from glycidyl (meth)acrylate as compared with 1,3-dichloro-2-propanol. The use of the resin avoids the inclusion of additional impurities into glycidyl (meth)acrylate and simplifies the process.

## Description

### FIELD OF THE INVENTION

The present invention relates to a process of purifying glycidyl (meth)acrylate by reducing the content of 1,3-dichloro-2-propanol in crude glycidyl (meth)acrylate obtained by the reaction of (meth)acrylic acid and epichlorohydrin.

### BACKGROUND OF THE INVENTION

Glycidyl (meth)acrylate is generally produced by the following three known methods using epichlorohydrin as a starting material:
(a) Reacting (meth)acrylic acid with epichlorohydrin in the presence of a quaternary ammonium salt to obtain 3-chloro-2-hydroxypropyl (meth)acrylate, which is then subjected to dehydrochlorination with alkali (Japanese Patent Publication No. 46-34010(1971) and Japanese Patent Application Laid-Open No. 48-5713(1973));
(b) Reacting (meth)acrylic acid with epichlorohydrin in the presence of a quaternary ammonium salt to obtain 3-chloro-2-hydroxypropyl (meth)acrylate, which is then subjected to ester interchange with an epoxy compound (Japanese Patent Publications Nos. 41-9005(1966) and 53-10575(1978), and Japanese Patent Application Laid-Open No. 50-95216(1975)); and
(c) Reacting (meth)acrylic acid with alkali to obtain alkali metal (meth)acrylate, which is then reacted with epichlorohydrin in the presence of a quaternary ammonium salt while eliminating alkali chloride (Japanese Patent Publications Nos. 45-28762(1970) and 48-4006(1973), and Japanese Patent Application Laid-Open No. 48-39423(1973)).

In any of the above known methods, the crude reaction solution contains 1,3-dichloro-2-propanol as an impurity. 1,3-Dichloro-2-propanol deteriorates the quality of glycidyl (meth)acrylate. In addition, since it contains harmful chlorine, it is desirable to remove 1,3-dichloro-2-propanol as completely as possible from the crude glycidyl (meth)acrylate in view of recent demands for environmental conservation.

The following problems are involved in obtaining high-quality glycidyl (meth)acrylate by distilling the crude reaction solution of glycidyl (meth)acrylate to remove impurities such as 1,3-dichloro-2-propanol. In the distillation using a packed distillation column, a pressure difference within the column raises the bottom temperature, this being likely to cause the polymerization of glycidyl (meth)acrylate at the bottom of the distillation column due to its high polymerizability. In addition, the polymerization of glycidyl (meth)acrylate may possibly occur when glycidyl (meth)acrylate is retained between packed materials in the distillation column. Such undesirable polymerization can be avoided by a simpler distillation. However, 1,3-dichloro-2-propanol is hardly removed from the crude glycidyl (meth)acrylate by simple distillations. Alternatively, it has been attempted to prevent glycidyl (meth)acrylate from being polymerized by suitably selecting the distillation conditions and polymerization inhibitors, etc. However, even when the polymerization can be prevented, the removal of 1,3-dichloro-2-propanol from the crude glycidyl (meth)acrylate is difficult by using a distillation column due to the closed boiling points, and quite a large number of distillation plates is required for sufficient removal.

Under these circumstances, many studies on production methods or purification methods have been hitherto made in order to obtain glycidyl (meth)acrylate with a low content of 1,3-dichloro-2-propanol.

It has been known that 1,3-dichloro-2-propanol is dehydrochlorinated and cyclized in the presence of alkali to form epichlorohydrin which can be readily separated from glycidyl (meth)acrylate by distillation (Japanese Patent Application Laid-Open (KOKAI) Nos. 7-2818(1995), 9-59268(1997) and 9-249657(1997)). However, the above method requires an additional water-washing step in order to remove the alkali and by-produced salts. When the washing with water is insufficient, the alkali not removed is introduced into the subsequent distillation step to likely cause hydrolysis of glycidyl (meth)acrylate, thereby increasing the content of glycidol in the collected glycidyl (meth)acrylate distillate and decreasing the yield of glycidyl (meth)acrylate.

Japanese Patent Application Laid-Open No. 4-187682(1992) discloses a method of removing a chlorine-containing compound from a crude glycidyl (meth)acrylate by stripping the crude glycidyl (meth)acrylate in the presence of a quaternary ammonium salt while blowing a mixed gas of oxygen and an inert gas thereinto. Japanese Patent Application Laid-Open No. 7-309854(1995) discloses a method of purifying glycidyl (meth)acrylate by heating a crude glycidyl (meth)acrylate in the presence of a quaternary ammonium salt and an alkali metal salt to distill off epichlorohydrin therefrom, filtering or washing with water the resultant mixture after adding sulfonic acid or a sulfonic acid salt, and then distilling the mixture. In these known methods, 1,3-dichloro-2-propanol is converted into epichlorohydrin and hydrogen chloride by the catalytic action of the quaternary ammonium salt, thereby facilitating the separation of impurities from the crude glycidyl (meth)acrylate. However, when the quaternary ammonium salt is present in the mixture being distilled, the amount of high-boiling components increases or the production of polymeric substances is accelerated, resulting in deterioration in workability and reduction in the yield of glycidyl (meth)acrylate. Therefore, complicated steps such as addition of a deactivating agent for the quaternary ammonium salt and filtration or water-washing for removing the quaternary ammonium salt are required before the distillation step.

As the method of producing glycidyl (meth)acrylate containing no 1,3-dichloro-2-propanol, are proposed ester interchange methods between methyl (meth)acrylate and glycidol in the presence of a basic catalyst (Japanese Patent Applications Laid-Open Nos. 47-18801(1972), 55-11542(1980), 55-102575(1980) and 6-1780(1994)). However, these methods involve problems such as low storage stability and high polymerizability of glycidol. In addition, methods of epoxidizing allyl (meth)acrylate are reported (Japanese Patent Publication No. 47-6289(1972), Japanese Patent Application Laid-Open Nos. 61-183275(1986), 5-92962(1993) and 6-116254(1994)). However, these methods are less economical due to the use of expensive raw materials and a large number of steps.

### SUMMARY OF THE INVENTION

It is an object of the present invention to provide a novel process for purifying glycidyl (meth)acrylate, which dissolves the above problems in the known methods.

As a result of extensive studies, the inventors have found that when a crude glycidyl (meth)acrylate containing 1,3-dichloro-2-propanol is contacted with a resin having ion exchange groups represented by the following formula (I): 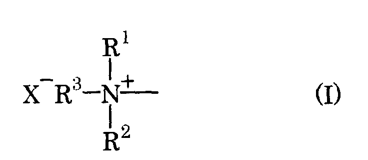 wherein R¹, R² and R³ are the same or different and are each H, HOC₂H₄, a C₁-C₁₀ alkyl group, a C₆-C₁₀ aryl group or a C₇-C₁₀ aralkyl group, and X is a halogen atom, 1,3-dichloro-2-propanol is effectively converted into epichlorohydrin which is readily separated from glycidyl (meth)acrylate by distillation, thereby enabling to produce high-quality glycidyl (meth)acrylate in industrially advantageous manner. The present invention has been attained on the basis of this finding.

Thus, in a first aspect of the present invention, there is provided a process for purifying glycidyl (meth)acrylate comprising a step of contacting a crude glycidyl (meth)acrylate containing 1,3-dichloro-2-propanol as an impurity with a resin having ion exchange groups represented by the formula (I): 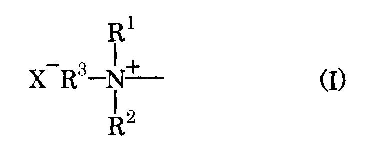 wherein R¹, R² and R³ are the same or different and are each H, HOC₂H₄, a C₁-C₁₀ alkyl group, a C₆-C₁₀ aryl group or a C₇-C₁₀ aralkyl group, and X is a halogen atom.

In a second aspect of the present invention, there is provided a process for purifying glycidyl (meth)acrylate comprising the steps of (1) contacting a crude glycidyl (meth)acrylate containing 1,3-dichloro-2-propanol as an impurity with a resin having ion exchange groups represented by the formula (I): 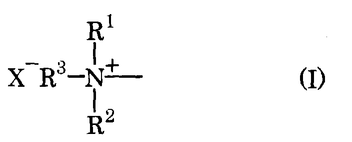 wherein R¹, R² and R³ are the same or different and are each H, HOC₂H₄, a C₁-C₁₀ alkyl group, a C₆-C₁₀ aryl group or a C₇-C₁₀ aralkyl group, and X is a halogen atom, to obtain a treated glycidyl (meth)acrylate; and (2) distilling the treated glycidyl (meth)acrylate to obtain a purified glycidyl (meth)acrylate.

### DETAILED DESCRIPTION OF THE INVENTION

Upon contacting a crude glycidyl (meth)acrylate containing 1,3-dichloro-2-propanol with the resin having ion exchange groups represented by the formula (I), 1,3-dichloro-2-propanol is converted into epichlorohydrin by the same catalytic action of the ion exchange groups as that of the quaternary ammonium salt. Unlike the use of the quaternary ammonium salt, the use of the resin does not cause the disadvantages such as contamination of glycidyl (meth)acrylate as well as decomposition and polymerization of glycidyl (meth)acrylate during distillation. For this reason, the use of the resin in place of the quaternary ammonium salt makes it unnecessary to add a deactivating agent for the quaternary ammonium salt and to conduct additional water-washing step for removing the quaternary ammonium salt before distillation. Further, the resin having ion exchange groups represented by the formula (I) can be continuously and repeatedly used as long as the catalytic activity is retained, making the process extremely economical.

The functional group represented by the formula (I) in the resin used in the present invention corresponds to a monovalent group derived from an ammonium halide by removing one hydrogen atom on the nitrogen atom. The ammonium halide may include monomethylammonium chloride, dimethylammonium chloride, trimethylammonium chloride, monoethylammonium chloride, diethylammonium chloride, triethylammonium chloride, tripropylammonium chloride, tributylammonium chloride, dimethylethanolammonium chloride, monomethyldiethanolammonium chloride, diethylethanolammonium chloride, monoethyldiethanolammonium chloride, dimethylbenzylammonium chloride, diethylbenzylammonium chloride, trimethylammonium bromide, triethylammonium bromide, trimethylammonium iodide, and triethylammonium iodide.

As the base resins to which the ion exchange groups represented by the formula (I) are bonded, any resins may be suitably used unless the use thereof adversely affects in practicing the present invention. The base resins are preferred to be resistant to any degradation upon contacting the components, such as glycidyl (meth)acrylate, 1,3-dichloro-2-propanol and epichlorohydrin, which are present in the process of the present invention. The base resins are further required to maintain mechanical properties when exposed to working temperatures, and cause no chemical change, such as deterioration in quality, of each component present in the process of the present invention.

In the present invention, basic anion exchange resins are particularly preferred as the resin having ion exchange groups represented by the formula (I). Examples of the basic anion exchange resins include Cl-type strongly-basic anion exchange resins having trimethylammonium ion exchange groups; Cl-type strongly-basic anion exchange resins having diethylethanolammonium ion exchange groups; hydrochloric acid-treated, weakly-basic anion exchange resins having primary or secondary amino ion exchange groups; and hydrochloric acid-treated, moderately-basic anion exchange resins having both of strongly-basic ion exchange groups and weakly-basic ion exchange groups.

As the base resins for the basic anion exchange resins, styrene-based resins and acrylic resins are preferable. The basic anion exchange resins may have any structure or configuration such as gel structure having no macropores, porous structure, high porous structure and MR structure having macropores. A structure having macropores is particularly preferable due to a fast conversion rate of 1,3-dichloro-2-propanol to epichlorohydrin.

The crude product to be treated in the present invention may be a crude glycidyl (meth)acrylate containing at least 1,3-dichloro-2-propanol or a crude liquid containing at least glycidyl (meth)acrylate and 1,3-dichloro-2-propanol, and these may be referred to simply as a crude glycidyl (meth)acrylate. The crude liquid may be a reaction product in known methods of producing glycidyl (meth)acrylate.

The step of contacting the crude glycidyl (meth)acrylate containing 1,3-dichloro-2-propanol with the resin having ion exchange groups represented by the formula (I) may be carried out in any stage after the production of glycidyl (meth)acrylate. However, at least most part of low-boiling components such as epichlorohydrin is preferably distilled off from the crude product prior to the contacting step. The contacting temperature should be regulated so as to proceed the conversion of 1,3-dichloro-2-propanol into epichlorohydrin and prevent the polymerization of glycidyl (meth)acrylate, and preferably from room temperature to 100°C. Further, the contacting temperature should be in a range where the resin having ion exchange groups represented by the formula (I) is stable. The resin may be used in an amount so that the total molar amount of the ion exchange groups is preferably 0.01 to 5 mol per one mol of 1,3-dichloro-2-propanol. The contacting step may be carried out in either batch-wise manner or continuous manner. Since the batch-wise manner requires subsequent recovery of the resin, the contacting step in continuous manner is preferable in industrial scale.

The crude glycidyl (meth)acrylate after subjected to the contacting step is distilled under reduced pressure in batch-wise or continuous manner to obtain purified glycidyl (meth)acrylate.

For example, the batch-wise purification of the crude glycidyl (meth)acrylate is carried out as follows. First, the crude glycidyl (meth)acrylate containing 1,3-dichloro-2-propanol is charged into a distillation still together with a polymerization inhibitor, and low-boiling components are distilled off under reduced pressure at a predetermined temperature. During the distillation, air or diluted air may be blown into the still to inhibit the polymerization of the crude glycidyl (meth)acrylate. To prevent the polymerization of glycidyl (meth)acrylate, the distillation for removing the low-boiling components is preferably carried out at a temperature as low as possible, and the pressure is correspondingly set to a lower level depending on the temperature employed. The pressure also depends upon the temperature of coolant and the ability of condenser to condense the low-boiling components. Usually, the temperature is 80°C or lower, and the pressure is 50 Torr or lower.

After an amount of the low-boiling components are distilled off, a suitable amount of the resin having ion exchange groups represented by the formula (I) is added into the still and the contents of the still are stirred while maintaining the still temperature within the range from room temperature to 100°C.

Thereafter, the resin is removed by filtration, and the filtrate is fed again into the distillation apparatus where the filtrate is distilled under a reduced pressure while blowing air or diluted air into the filtrate. After separating the low-boiling components from the filtrate as an initial distillate, the distillate of the aimed glycidyl (meth)acrylate is collected. During the distillation of glycidyl (meth)acrylate, a polymerization inhibitor or glycidyl (meth)acrylate dissolving a polymerization inhibitor may be added to the top of distillation column and the condenser. The actual conditions for distillation such as temperature, pressure, etc. may be easily determined depending on the factors familiar to those skilled in the art.

For example, the continuous purification of the crude glycidyl (meth)acrylate is carried out as follows. First, the crude glycidyl (meth)acrylate containing 1,3-dichloro-2-propanol is continuously supplied to a distillation column together with a polymerization inhibitor, and heated there at a temperature of 80°C or lower under a reduced pressure of 50 Torr or lower to distill off most part of the low-boiling components from the top of the distillation column while taking out a crude glycidyl (meth)acrylate from the bottom.

The crude glycidyl (meth)acrylate taken out of the bottom of the distillation column is allowed to pass through a column packed with the resin having ion exchange groups represented by the formula (I) at room temperature to 100°C. The packed length, the flow rate and the number of repetition of the passing are determined such that 1,3-dichloro-2-propanol may contact the resin for a sufficient period of time effective for converting 1,3-dichloro-2-propanol to epichlorohydrin.

The resultant glycidyl (meth)acrylate from the contacting step is supplied to a distillation column to distill off the low-boiling components from the top of the column, while removing the high-boiling components from the bottom and collecting the purified glycidyl (meth)acrylate as a side-cut.

Alternatively, the low-boiling components may be distilled off from the top of the column, while taking out a crude glycidyl (meth)acrylate containing the high-boiling components from the bottom, which is then continuously supplied to a next distillation column where the purified glycidyl (meth)acrylate is collected from the top of the column and the high-boiling components are removed from the bottom.

In the above continuous purification method, air or diluted air may be blown into any of the distillation columns to prevent the polymerization of glycidyl (meth)acrylate. Also, the polymerization inhibitor may be add directly or in the form of a solution in glycidyl (meth)acrylate into the column or the top thereof, or into the condenser. The polymerization inhibitors may include phenol polymerization inhibitors such as methoxyphenol, and amine polymerization inhibitors such as N-isopropyl-N'-phenyl-p-phenylenediamine.

By the purification process of the present invention, the following advantageous effects are obtained:
(1) Since no further impurities such as quaternary ammonium salt contaminate glycidyl (meth)acrylate, additional steps such as water-washing and filtration for removing such impurities are not required. Further, the amount of 1,3-dichloro-2-propanol is easily and efficiently reduced to about 0.2% by weight or less, preferably 0.12% by weight or less based on glycidyl (meth)acrylate only by contacting the crude glycidyl (meth)acrylate with the resin having ion exchange groups represented by the formula (I), for example, only by passing the crude glycidyl (meth)acrylate through a column packed with the resin. This enables the production of glycidyl (meth)acrylate with a high quality in an industrially advantageous process.
(2) Since no further impurities contaminate a distillation mother liquor, glycidyl (meth)acrylate is effectively prevented from polymerization at the bottom of the distillation column, thereby avoiding to take a large amount of still residue and resulting in increased yield and productivity of the aimed product.
(3) Since the amount of 1,3-dichloro-2-propanol, which is extremely difficult to separate from glycidyl (meth)acrylate, is reduced, the distillation column is not required to have a high separation ability. In addition, since the polymerization of glycidyl (meth)acrylate due to the bottom temperature rise caused by the pressure difference and the polymerization due to the retention of glycidyl (meth)acrylate between the packing materials are effectively prevented, the troubles which considerably deteriorate productivity of the aimed product are avoided. This results in high productivity of the aimed product.

The present invention will be described in detail below by way of examples and comparative examples. Each component was identified and the amount thereof was determined by gas chromatographic analysis under the following conditions:
Column: capillary column PEG-20M (DB WAX) manufactured by J & W Co., Ltd.
Injection temperature: 230°C
Column temperature: raised from 100°C to 230°C at a rate of 4°C/min.
Detector temperature: 230°C

### Example 1

A strongly basic anion exchange resin (Cl-type) Amberlist A26 (trade name, produced by Organo Co., Ltd.) was washed with ion-exchanged water and then with methanol to replace the water, and dried in vacuum at 40°C for 5 hours.

Into a 200cc round bottom flask, were charged 100 g of a crude glycidyl methacrylate containing 0.8 % by weight of 1,3-dichloro-2-propanol, and 50 ppm of methoxyphenol as a polymerization inhibitor (produced by Kawaguchi Chemical Co., Ltd.). Into the flask, 5 g of the above anion exchange resin were further added. The contents of the flask were stirred at 80°C for one hour. The resultant mixture was filtered to remove the resin, and the resultant filtrate was subjected to gas chromatographic analysis. The concentrations of 1,3-dichloro-2-propanol in glycidyl methacrylate before and after contacting the anion exchange resin are shown in Table 1 as area ratios corrected for by taking factors into account.

### Example 2

A strongly basic anion exchange resin (Cl-type) Diaion HPA75 (trade name, produced by Mitsubishi Chemical Corp.) was washed with ion-exchanged water and then with methanol to replace the water, and dried in vacuum at 40°C for 5 hours.

Into a 200cc round bottom flask, were charged 100 g of the same crude glycidyl methacrylate as used in Example 1. Into the flask, 5 g of the above anion exchange resin were further added. The contents of the flask were stirred at 60°C for one hour. The resultant mixture was filtered to remove the resin, and the resultant filtrate was subjected to gas chromatographic analysis. The concentrations of 1,3-dichloro-2-propanol in glycidyl methacrylate before and after contacting the anion exchange resin are shown in Table 1 as area ratios corrected for by taking factors into account.

### Example 3

A weakly basic anion exchange resin (amine-type) Amberlist A21 (trade name, produced by Organo Co., Ltd.) was washed with ion-exchanged water and then suspended in 250cc of 0.2 mol/liter hydrochloric acid per 4 cc of the anion exchange resin while stirring at room temperature for 8 hours. Thereafter, the anion exchange resin was sufficiently washed with ion-exchanged water until the washing became neutral and then with methanol to replace the water, and dried in vacuum at 40°C for 5 hours.

Into a 200cc round bottom flask, were charged 100 g of the same crude glycidyl methacrylate as used in Example 1. Into the flask, 5 g of the above anion exchange resin were further added. The contents of the flask were stirred at 60°C for three hours. The resultant mixture was filtered to remove the resin, and the resultant filtrate was subjected to gas chromatographic analysis. The concentrations of 1,3-dichloro-2-propanol in glycidyl methacrylate before and after contacting the anion exchange resin are shown in Table 1 as area ratios corrected for by taking factors into account.

**Table 1**

| | Cl-type basic anion exchange rein | Contacting conditions (hour/°C) | Concentration of 1,3-DCP*¹ (wt. %) |
|---|---|---|---|
| Before contacting | - | - | 0.80 |
| Example 1 | Amberlist A26 | 1/80 | 0.03 |
| Example 2 | Diaion HPA75 | 1/60 | 0.01 |
| Example 3 | Amberlist A21 | 3/60 | 0.11 |

| | | | |
|---|---|---|---|
| Note: *1) 1,3-dichloro-2-propanol | | | |

As seen from Table 1, the concentration of 1,3-dichloro-2-propanol in glycidyl methacrylate was easily reduced by merely contacting the resin having ion exchange groups represented by the formula (I). In addition, it was recognized that many types of groups were effective as the ion exchange groups represented by the formula (I).

### Example 4

Into a SUS316 cylindrical column having an inner diameter of 21 mm, were packed 8 g of a strongly basic anion exchange resin (Cl-type) Diaion HPA25 (trade name, produced by Mitsubishi Chemical Corp.). The same crude glycidyl methacrylate as used in Example 1 was allowed to continuously flow upwardly through the cylindrical column at a flow rate of 0.9 g/min at 66°C. The total amount of glycidyl methacrylate passed through the column was 4,000 times the amount of the packed resin. From the beginning to the completion of the contacting operation, the concentration of 1,3-dichloro-2-propanol in the effluent glycidyl methacrylate from the column was kept constant at 0.01 % by weight. The amount of the ion exchange groups represented by the formula (I) in the packed resin was 0.018 mol in total, and the total reduced amount of 1,3-dichloro-2-propanol was 2.4 mol. Therefore, it was clear that the ion exchange groups in the anion exchange resin acted as a catalyst for converting 1,3-dichloro-2-propanol into epichlorohydrin.

### Example 5

Into a 1,000cc conical flask, were charged 500 g of a crude reaction liquid obtained from the reaction of methacrylic acid and epichlorohydrin in the presence of sodium carbonate. The chemical composition of the crude reaction liquid is shown in Table 2. To the flask, 25 g of Cl-type Diaion HPA25 were further added. The contents of the flask were stirred for one hour while heating at 60°C over oil bath. Thereafter, the resultant mixture was filtered to remove the resin.

The filtrate thus obtained was subjected to distillation in the following manner using a distillation apparatus having a 500cc three-necked flask equipped with a thermometer and a capillary for feeding air, a packed column with ring packing materials equivalent to four plates, a cooling tube, a vacuum trap cooled with methanol/dry ice and a vacuum pump.

Into the three-necked flask, were charged 400 g of the crude reaction liquid after contacting the anion exchange resin, and 0.8 g of N-isopropyl-N'-phenyl-p-phenylenediamine as a polymerization inhibitor (Antage 3C (trade name) produced by Kawaguchi Chemical Industry Co., Ltd.). The mixture was heated in oil bath from room temperature to 80°C while maintaining the top pressure of the column at 6 Torr. After epichlorohydrin was completely distilled, the top pressure of the column was reduced to 4 Torr and the temperature of the oil bath was raised to 100°C to further continue the distillation. The initial distillate was rejected until the total amount of the distilled epichlorohydrin, the content collected in the vacuum trap and the initial cut reached about 30 % by weight of the crude reaction liquid, and then the subsequent distillate was collected until about 80 % by weight of the crude reaction liquid was distilled. After the distillation, no polymeric still residue was noticed. The temperature of the liquid phase during the distillation of glycidyl methacrylate was 82 to 90°C, and the top temperature of the distillation column was 68°C. The collected distillate was subjected to gas chromatographic analysis. The results expressed by simple area ratios are shown in Table 2.

### Comparative Example 1

Into a three-necked flask, were charged 400 g of the same crude reaction liquid, before contacting Cl-type Diaion HPA25, as used in Example 5 and 0.8 g of N-isopropyl-N'-phenyl-p-phenylenediamine. Then, the mixture was subjected to simple distillation using the same distillation apparatus under the same distillation conditions as in Example 5. The initial distillate was rejected until the total amount of the distilled epichlorohydrin, the content collected in the vacuum trap and the initial cut reached about 30 % by weight of the crude reaction liquid, and then the subsequent distillate was collected until about 80 % by weight of the crude reaction liquid was distilled. After the distillation, no polymeric still residue was noticed. The collected distillate was subjected to gas chromatographic analysis. The results expressed by simple area ratios are shown in Table 2.

**Table 2**

| | Results of gas chromatographic analysis | | |
|---|---|---|---|
| | EpCH*² | GMA*³ | 1,3-DCP*¹ |
| Crude reaction liquid | 13.49 | 74.91 | 0.327 |
| Example 5 | 0.018 | 99.67 | 0.005 |
| Comparative Example 1 | 0.030 | 99.35 | 0.397 |

| | | | |
|---|---|---|---|
| Note: *1) 1,3-dichloro-2-propanol | | | |
| *2) epichlorohydrin | | | |
| *3) glycidyl methacrylate | | | |

As seen from Table 2, the content of 1,3-dichloro-2-propanol, which was extremely difficult to separate from glycidyl (meth)acrylate, was considerably reduced by merely contacting the crude reaction liquid of glycidyl methacrylate with the resin having ammonium salt groups, thereby enhancing the purity of glycidyl methacrylate. Further, during the distillation, glycidyl methacrylate was completely prevented from polymerizing at the bottom of the distillation column.

## Claims

1. A process for purifying a crude glycidyl (meth)acrylate comprising a step of contacting the crude glycidyl (meth)acrylate containing 1,3-dichloro-2-propanol as impurity, with a resin having ion exchange groups represented by the following formula (I): 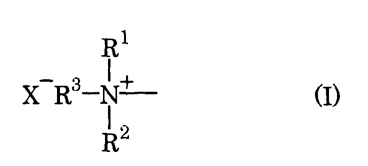 wherein R¹, R² and R³ are the same or different and are each H, HOC₂H₄, a C₁-C₁₀ alkyl group, a C₆-C₁₀ aryl group or a C₇-C₁₀ aralkyl group, and X is a halogen atom.

2. The process according to claim 1, wherein said crude glycidyl (meth)acrylate after contacting the resin is further purified by distillation.

3. The process according to claim 1 or 2, wherein said resin having the ion exchange groups represented by the formula (I) is a Cl-type basic anion exchange resin.

4. The process according to claim 3, wherein said Cl-type basic anion exchange resin has a highly-porous structure having macropores.

5. The process according to any one of claims 1 to 4, wherein said crude glycidyl (meth)acrylate is contacted with the resin at room temperature to 100°C.

6. The process according to any one of claims 1 to 5, wherein said resin is used in an amount such that a total amount of the ion exchange groups is 0.01 to 5 mol per one mol of 1,3-dichloro-2-propanol.

7. The process according to any one of claims 1 to 6, wherein said crude glycidyl (meth)acrylate is subjected to a pre-treatment prior to contacting the resin having the ion exchange groups represented by the formula (I) to distill off most part of low-boiling components.

8. The process according to claim 7, wherein said pre-treatment is carried out at 80°C or lower under a pressure of 50 Torr or lower.

9. The process according to any one of claims 1 to 8, wherein a content of 1,3-dichloro-2-propanol in glycidyl (meth)acrylate is reduced to 0.2 % by weight or less.
